# EUROPEAN PATENT APPLICATION

(11) **EP 2 698 170 A1**
(43) Date of publication of application: **19.02.2014**
(21) Application number: 13192890.5
(22) Date of filing: 21.06.2006
(51) Int. Cl.: A61K 49/00, A61K 51/00, A61K 51/04

(54) **Use of DTBZ for imaging endocrine pancreas and beta cell mass in type 1 diabetes**

(30) Priority: 29.06.2005 US 695741 P
(62) Divisional of application: 06773643.9
(71) Applicant: The Trustees of Columbia University in the City of New York, New York, NY 10027 (US)
(72) Inventor: Harris, Paul, New York, NY 10033 (US); Maffei, Antonella, New York, NY 10033 (US); Van Heertum, Ronald, Morristown, NJ 07960 (US)
(74) Representative: Schiener, Jens

(57) **Abstract**

The present invention provides non-invasive methods for determining the beta cell mass in the pancreas of a subject by administering to the subject an effective amount of a vesicular monoamine transporter type 2 (VMAT2)-specific radioligand; obtaining at least one computerized image of at least a portion of the pancreas of the subject; and quantitatively analyzing the computerized image in order to determine the beta cell mass in the pancreas of the subject. The present invention additionally provides methods for diagnosing a metabolic neuroendocrine disorder in a subject including insulinoma, diabetes and preclinical diabetes, as well as methods for determining the efficacy of treatment for a metabolic disorder. The present invention further provides methods for assessing the prognosis of a subject at risk for developing diabetes, and methods for managing the treatment or prevention of diabetes. The present invention also provides kits for use in determining the beta cell mass in the pancreas of a subject, as well as diagnosing metabolic or neuroendocrine disorders in a subject.

## Description

### STATEMENT OF GOVERNMENT INTEREST

This invention was made with government support under NIH Grant No. RO1 DK 63567-02. As such, the United States government has certain rights in this invention.

### FIELD OF THE INVENTION

This invention relates to non-invasive methods for quantitatively measuring beta cell mass in a subject useful in the management of metabolic disorders including diabetes.

### BACKGROUND OF THE INVENTION

Type 1 Diabetes (T1D) is a result of autoimmune destruction of the insulin-producing beta cells of the islets of Langerhans, the endocrine component of the pancreas (Weir, et al, 1990, Islet mass and function in diabetes and transplantation, Diabetes 39:401-405). This disease has an insipid beginning and may take years to be recognized. While it is generally thought that the majority of the mass of beta cells is destroyed at the time of presentation with diabetes, several recent studies have suggested that there may be significant residual insulin secretory capacity at diagnosis (Steele, et al, 2004, Insulin secretion in type 1 diabetes, Diabetes 53:426-433). Moreover, there is a long preclinical period during which an immunologic assault is believed to occur on the islets of Langerhans and that.hyperglycemia only develops when a critical mass of beta cells is lost, and insulin requirements increase.

The natural history of T1D is progression to complete elimination of insulin secretory capacity and dependence on exogenous insulin for survival. However, it has not been possible to accurately determine the Beta Cell Mass (BCM) that is present in individuals with diabetes and therefore, conclusions about the natural history as well as effects of newer interventions on this process are based on indirect evidence. Similarly, a number of abnormalities in the insulin producing capacity of the pancreas have been described for patients with type 2 diabetes (T2D) (Bernard-Kargar, et al, 2001, Endocrine pancreas plasticity under physiological and pathological conditions, Diabetes 50 Suppl 1:530-35), but there does not exist a means of measuring BCM that would allow differentiation of functional versus anatomical defects in insulin secretion in this form of the disease.

A variety of experimental treatments have been developed to treat T1D including immunotherapy, stem cell therapy and islet transplantation. The treatment of T2D has been largely empirical due to the lack of understanding of the basic mechanisms at work in the disease. An understanding of how the beta cell mass changes during the various phases of diabetes will provide important information to help investigators develop new therapies for both types of diabetes.

Progress towards imaging disease of the endocrine pancreas has been described in several studies. Clark, *et al.,* demonstrated that the body of the pancreas can be imaged with fluorine-18 4-fluorobenzyltrozamicol (FBT), a radioligand that binds to specific neuroreceptors, vesicular acetylcholine transporters, present on presyntaptic vesicles in the neurons innervating the pancreas (Clark, et al, 2003, Neurofunrtional imaging of the pancreas utilizing the cholinergic PET radioligand [(18)F]4-fluorobenzyltrozamicol, Eur J Nucl Med Mol Imaging). Similarly, taking advantage of the bicarbonate and/or organic anion transporters expressed by pancreatic acinar cells, [11C] acetate has been used to visualize the exocrine pancreas (Shreve, et al, 1997, Imaging of the pancreas and related disease with PET carbon-11-acetate, J Nucl Med 38:1305-1310. and Seltzer, et al., 2004, Radiation dose estimates in humans for (11)C-acetate whole-body PET, J Nucl Med 45:1233-1236). Markmann, *et al.,* recently reported that transplanted cadaveric islets, 14 months post transplantation, induces peri-islet cell mass fat deposits that are visible by chemical shift gradient-echo magnetic resonance imaging (MRI) (Markmann, et al, 2003, Magnetic resonance-defined periportal steatosis following intraportal islet transplantation: a functional footprint of islet graft survival? Diabetes 52:1591-1594). A possible problem with this approach is that peri-islet steatosis is likely to persist, at least for a few days, following islet allograft rejection and the method is not suitable for imaging islets *in situ.*

Other previous attempts to image β-cells and T1D related pathology include the studies by Moore, *et al.* (Moore, et al, 2001, Noninvasive in vivo measurement of beta-cell mass in mouse model of diabetes, Diabetes 50:22312236, Moore, et al, 2004, Tracking the recruitment of diabetogenic CD8+ T-cells to the pancreas in real time, Diabetes 53:1459-1466, and Moore, et al, 2002, MRI of insulitis in autoimmune diabetes, Magn Reson Med 47:751-758). Using a beta cell specific anti IC2 mAb, modified with a radioisotope chelator, normal and diabetic rodent pancreata were imaged *ex vivo.* Radioimmunoscintigraphy showed major differences in pancreatic uptake of the mAb between normal and diabetic rodents (Moore, et al, 2001, Noninvasive in vivo measurement of beta-cell mass in mouse model of diabetes, Diabetes 50:22312236), but it was unclear if the method was suitable for *in vivo* imaging. Radioimmunoscintigraphy with antiganglioside mAbs have been less promising (Ladriere, et al, 2001, Pancreatic fate of a (125)1-labelled mouse monoclonal antibody directed against pancreatic B-cell surface ganglioside(s) in control and diabetic rats, Cell Biochem Funct 19:107-115).

In other studies the uptake 6-deoxy-6-[1251]iodo-D-glucose by pancreata from normal versus streptozotocin-injected rats has been compared. Although islets and acinar tissue showed differential uptake of the radioligand, and beta cell depleted pancreata showed decreased uptake, the clinical utility of this approach is unclear because of the broad specificity of binding of the radioligand and high uptake in the liver (Malaisse, et al, 2001, Pancreatic uptake of [2-(14)C] alloxan, Int J Mol Med 7:311-315). Pancreatic uptake of a tracer [2-(14)C] alloxan has been studied in normal and streptozotocintreated rodents. Preferential uptake of the radiotracer in normal versus the diabetic pancreas was demonstrated. Alloxan, however, is a well known diabetogenic agent itself and thus the clinical utility of this approach remains unproven (Malaisse, et al, 2001, Pancreatic uptake of [2-(14)C]alloxan, Int J Mol Med 7:311-315). Dithizone and Sulfonylurea receptor ligands [e.g., 3H-glibenclamide] have been studied as possible imaging agents, but show broad tissue distributions of uptake counterindicating feasibility (Garnuszek, et al, 2000, Identification of transplanted pancreatic islet cells by radioactive dithizone-[1 311] -histamine conjugate. Preliminary report, Nucl Med Rev Cent East Eur 3:61-63. Ladriere, et al, 2000, Uptake of tritiated glibenclamide by endocrine and exocrine pancreas, Endocrine 13:133-136, and Sweet, et al, 2004, Systematic screening of potential beta-cell imaging agents, Biochem Biophys Res Commun 314:976-983).

The use of magnetic resonance imaging has been explored in experimental insulitis. Moore, *et al.,* using superparamagnetic particle labeled T cells (via a CLIO-TAT peptide or MHC tetramer peptide complexes), were able to clearly demonstrate the presence of infiltrating T cells during the evolution of beta cell destruction (Moore, et al, 2004, Tracking the recruitment of diabetogenic CD8+ T-cells to the pancreas in real time, Diabetes 53:1459-1466, and Moore, et al, 2002, MRI of insulitis in autoimmune diabetes, Magn Reson Med 47:751-758) Magnetic resonance imaging has also been used to visualize peri-islet vascular leakage due to insulitis using superparamagnetic nanobeads (Denis, et al, 2004, Imaging inflammation of the pancreatic islets in type 1 diabetes, Proc Natl Acad Sci USA 101:12634-12639).

Despite different embryological origins, β-cells of the endocrine pancreas and neurons share expression of a large number of gene products and display many functional similarities. Previous studies, at both protein and nucleic acid levels, have shown the underlying physiochemical basis for this functional similarity (Atouf, et al, 1997, Expression of neuronal traits in pancreatic beta cells. Implication of neuron-restrictive silencing factor/repressor element silencing transcription factor, a neuron-restrictive silencer, J Biol Chem 272:1929-1934, Bernal-Mizrachi, et al, 2003, Gene expression profiling in islet biology and diabetes research, Diabetes Metab Res Rev 19:32-42, and Scharfinann, et al, 1996, Differentiation and growth of pancreatic beta cells, Diabetes Metab 22:223-228). The inventors own gene expression mapping studies led them to focus on one such shared gene product, VMAT2, vesicular monoamine transporter type 2, expressed by β-cells, but absent from the exocrine pancreas and a variety of-the other abdominal organs (Maffei, et al, 2004, Identification of tissue-restricted transcripts in human islets, Endocrinology 145:4513-4521). A specific ligand for VMAT2, dihydrotetrabenazine (DTBZ) is already in clinical use for positron emission tomography (PET) imaging of central nervous system disorders (Vander Borght, et al, 1995, In vivo imaging of the brain vesicular monoamine transporter, J Nucl Med 36:2252-2260). The inventors studied the binding of [3H]DTBZ to total membranes fractions prepared from purified human islets and purified exocrine pancreas tissue. The inventors found [3H]DTBZ specifically bound to islet membranes but not to membranes from the exocrine pancreas. Immunohistochemistry further showed that anti VMAT2 and insulin immunoreactivity co-localized in islet β-cells (Weihe, et al, 2000, Chemical neuroanatomy of the vesicular amine transporters, Faseb J 14:2435-2449, Weihe, et al, 1994, Localization of vesicular monoamine transporter isoforms (VMAT1 and VMAT2) to endocrine cells and neurons in rat, J Mol Neurosci 5:149-164, Anlauf, et al, 2003, Expression of the two isoforms of the vesicular monoamine transporter (VMAT1 and VMAT2) in the endocrine pancreas and pancreatic endocrine tumors, J Histochem Cytochem 51:1027-1040. and Mei, et al, 2002, Early, selective and marked loss of sympathetic nerves from the islets of BioBreeder diabetic rats, Diabetes 51:2997-3002).

In view of the foregoing, there is a need to identify new methods for non-invasive determination of beta cell mass (BCM), which can be used, for example to provide a quantitative endpoint for therapy of diabetes, islet regeneration and transplantation. The inventors disclose herein that quantitation of VMAT2 expression in beta cells by use of [11C] DTBZ and PET provides such a method for non-invasive measurements of B CM. More particularly, the inventors disclose that [11C] DTBZ can be used to image the endocrine pancreas *in vivo* and that PET imaging with this radioligand could discriminate euglycemic rats from rats with spontaneous diabetes or diabetes induced by streptozotocin (STZ).

### SUMMARY OF THE INVENTION

The present invention is based on the discovery that the beta cell mass of the endocrine pancreas can be readily and non-invasively visualized and quantitatively measured using computer tomography (CT), and that this method provides support for new experimental therapies, as well as monitoring and.management of beta cell associated disorders including diabetes. Importantly, this method provides a diagnostic test for nascent diabetes in individuals at risk.

Accordingly, in one aspect, the present invention provides methods for determining the beta cell mass in the pancreas of a subject by administering to the subject an effective amount of a vesicular monoamine transporter type 2 (VMAT2) - specific radioligand; obtaining one or more computerized image(s) of at least a portion of the pancreas of the subject; and quantitatively analyzing the computerized image(s) in order to determine the beta cell mass in the pancreas of the subject. In one embodiment of the present invention, the VMAT2-specific radioligand is [11C] dihydrotetrabenazine ([11C] DTBZ). In another embodiment of the present invention, the computerized image is obtained using a positron emission tomography (PET). Although methods of the present invention can be used to determine the beta cell mass in any mammalian subject, the subject is preferably human.

The present invention also provides methods for diagnosing a metabolic or neuroendocrine disorder in a subject by administering to the subject an effective amount of a VMAT2-specific radioligand; obtaining one or more computerized image(s) of at least a portion of the pancreas of the subject; quantitatively analyzing the computerized image(s) in order to determine the beta cell mass in the pancreas of the subject; and comparing the beta cell mass with a baseline measure of beta cell mass, where decreased beta cell mass or increased beta cell mass versus the baseline measure is associated with the presence of a metabolic or neuroendocrine disorder. In one embodiment of the invention, the radioligand is [11C] DTBZ and the computerized image is obtained using PET. In another embodiment of the invention, the metabolic disorder is a beta cell associated disorder, including but not necessarily limited to, an insulinoma or other neuroendocrine tumor. In another embodiment, the beta cell associated disorder is diabetes. In a particular embodiment of the invention, the metabolic disorder is type 1diabetes. In another embodiment, the metabolic disorder is type 2 diabetes. In a further embodiment, the metabolic disorder is preclinical type 1 diabetes.

The present invention additionally provides methods for assessing the prognosis of a subject at risk for developing diabetes by periodically administering to the subject an effective amount of a VMAT2-specific radioligand; obtaining one or more computerized image(s) of at least a portion of the pancreas of the subject; quantitatively analyzing the computerized image(s) in order to determine the beta cell mass in the pancreas of the subject; and comparing the periodically determined beta cell mass with a baseline measure of beta cell mass, where decreased beta cell mass versus the baseline measure is associated with the progression from a prediabetic condition to a diabetic condition. In an embodiment of the invention, the radioligand is [11C] DTBZ and the computerized image is obtained using PET. In another embodiment of the present invention, the subject is at risk for developing type 1 or type 2 diabetes.

The present invention also provides methods for determining the efficacy of therapy of a metabolic or disorder by periodically administering to the subject an effective amount of a VMAT2-specific radioligand; obtaining one or more computerized image(s) of at least a portion of the pancreas of the subject; quantitatively analyzing the computerized image(s) in order to determine the beta cell mass in the pancreas of the subject; and comparing the periodically determined beta cell mass with a baseline measure of beta cell mass, where a beta cell mass generally equivalent to the baseline measure is indicative of successful therapy to treat the metabolic disorder. In an embodiment of the invention, the radioligand is [11C] DTBZ and the computerized image is obtained using PET. In a further embodiment of the invention, the metabolic disorder is a pancreatic beta cell associated disorder. In a specific embodiment, the beta cell disorder is an insulinoma or a neuroendocrine tumor. In yet another specific embodiment, the beta cell disorder is diabetes. In still other embodiments of the invention, the beta cell disorder is type 1 diabetes, type 2 diabetes or preclinical type 1 diabetes.

The invention also provides methods for managing the treatment or prevention of diabetes by periodically administering to the subject an effective amount of a VMAT2-specific radioligand; obtaining one or more computerized image(s) of at least a portion of the pancreas of the subject; quantitatively analyzing the computerized image(s) in order to determine the beta cell mass in the pancreas of the subject; and comparing the periodically determined beta cell mass with a baseline measure of beta cell mass, where decreased beta cell mass versus the baseline measure is associated with the need for further therapy. In an embodiment of the invention, the radioligand is [11C] DTBZ and the computerized image is obtained using PET. In one embodiment, the diabetes is type 2 diabetes. In a preferred embodiment, the diabetes is type 1 diabetes.

The invention also provides kits for use in determining the beta cell mass in the pancreas of a subject and for detecting diabetes in a subject comprising an effective amount of VMAT2-specific radioligand, a pharmaceutically acceptable carrier, and optionally, a PET scanner. In an embodiment, the radioligand is [11C] DTBZ. In another embodiment of the invention, the diabetes is type 1 diabetes or type 2 diabetes.

The present invention further provides method for diagnosing a neuroendocrine disorder in a subject comprising administering to the subject an effective amount of a vesicular monoamine transporter type 2 (VMAT2)-specific radioligand; obtaining at least one computerized image of at least a portion of a region of interest of the subject; quantitatively analyzing the computerized image in order to determine the beta cell mass in the region of interest of the subject; and comparing the beta cell mass with a baseline measure of beta cell mass, where a decreased beta cell mass or increased beta cell mass versus the baseline measure is associated with the presence of a neuroendocrinedisorder. In one embodiment of the invention, the neuroendocrine disorder is a neuroendocrine cancer, such as prostate cancer.

The present invention also provides methods for imaging a neuroendocrine tumor by administering to the subject an effective amount of a vesicular monoamine transporter type 2 (VMAT2)-specific radioligand; and obtaining at least one computerized image of at least a portion of a region of interest of the subject. In one embodiment, the tumor is a prostate tumor.

Other feature and advantages of the present invention will become apparent from the following detailed description. It should be understood, however, that the detailed description and the specific examples while indicating the preferred embodiments of the invention are given by way of illustration only, since various changes and modifications within the spirit and scope of the invention will become apparent to those skilled in the art from this detailed description.

### DESCRIPTION OF THE FIGURES

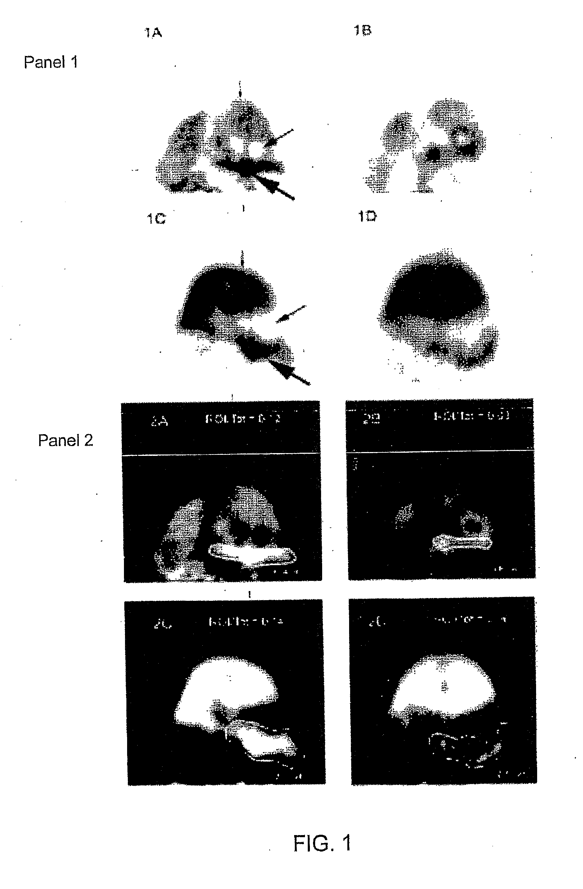

FIG. 1 shows representative coronal planes of PET images of abdomens of normal (Panels 1 A and 1 C) and STZ induced diabetic (Panels 1 B and 1 D) rats obtained with [11 C] DTBZ. The body of endocrine pancreas is identified by large arrows. The left lobe of the liver (small arrows) and the void of the stomach (medium arrows) are also identified. Approximately 300 µCi of (+)-α-[11C] DTBZ were used for imaging. Normal Lewis rats were imaged followed by induction of diabetes and then imaged a second time. Blood glucose concentrations of animals imaged in panels A and C ranged from 80 to 120 mg/dl, blood glucose levels of animals imaged in panels B and D were greater than 300 mg/dl. Data presented is the summed image of the scanning period. Animal imaged in panels A and B were fasted six hours prior to imaging, animal imaged in C and D fed *ad libitum.* Quantitation of activity within the region of interest (Panels 2A-D) showed reduced (approximately 20-40%) [11C]DTBZ uptake following STZ treatment relative to the total activity in the field. The integrated grey scale density value for each field appears in the lower left hand corner of panels 2 A-D. The top of the figure is rostral.

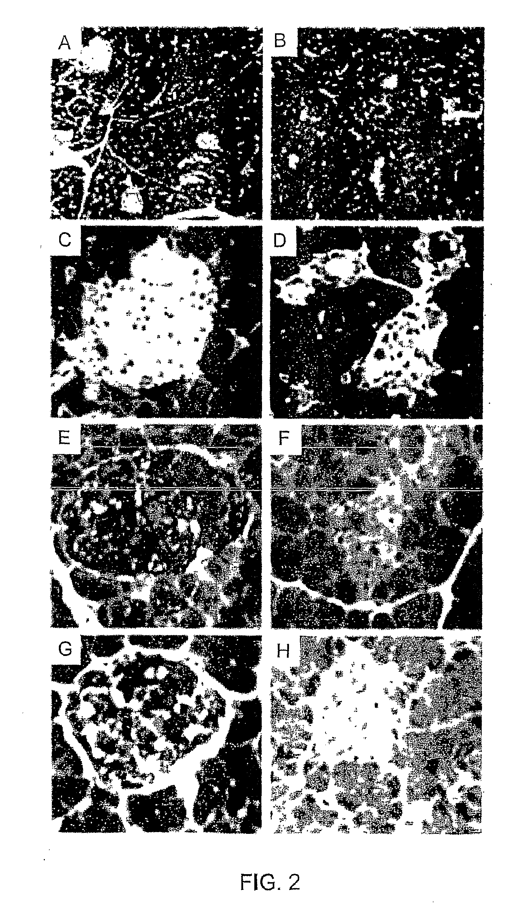

FIG. 2 shows Immunohistochemistry of pancreata from euglycemic controls (Left Panels) and STZ-induced diabetic Lewis rats (Right Panels) following imaging. Paraffin embedded sections from control and STZ treated rat pancreata were stained by H&E (Panels A-D) or processed for immunohistochemistry with anti insulin antibodies (Panel E and F) or antibodies to VMAT2 (Panel G and H). Sections in the right panels originated from the body of the pancreas of diabetic rats where loss of DTBZ binding was observed. Indirect staining of slides was performed with horseradish peroxidase conjugated anti goat or guinea pig Igs and developed with DAB. Panels A and B are photomicrographs at 100x magnification, all other panels taken at 400x magnification.

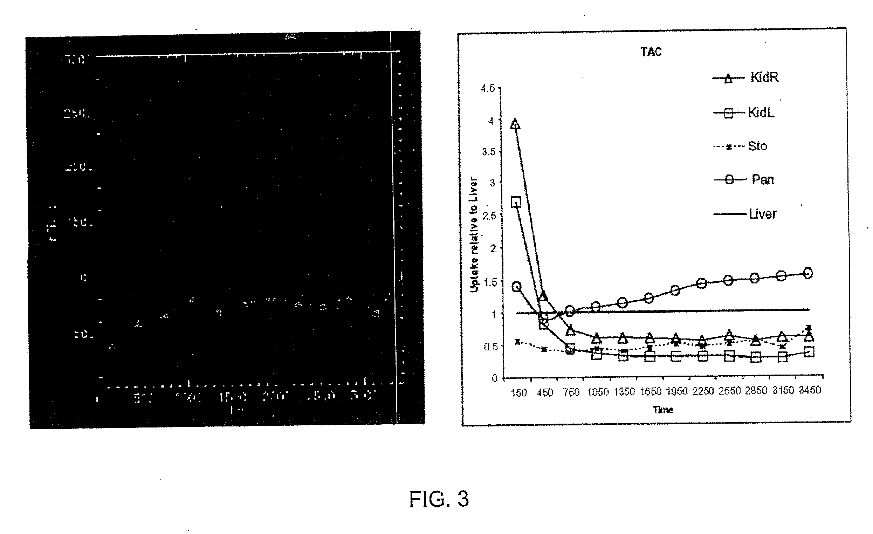

FIG. 3 shows time activity curves (TAC) for first scan (racemic DTBZ) in a normal rat.

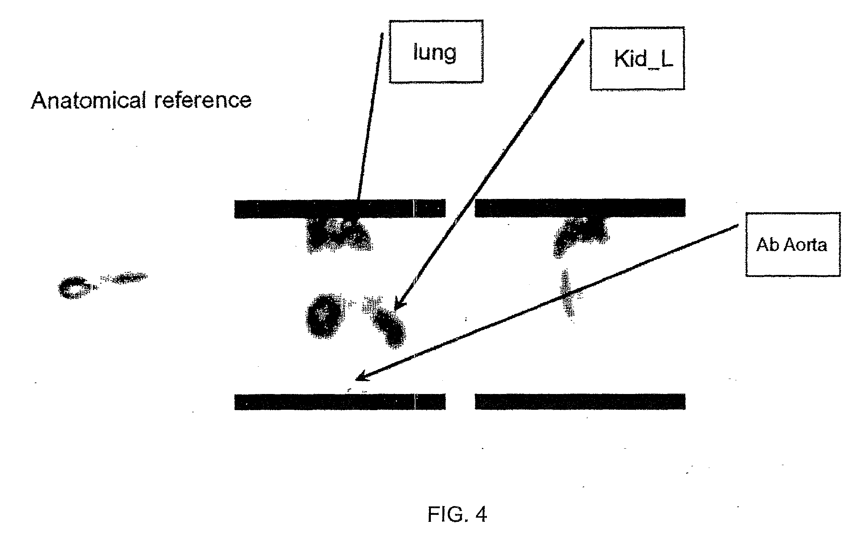

FIG. 4 shows the summed frames of the first 4 minutes of the PET scan of Bob 19 (normal Lewis rat).

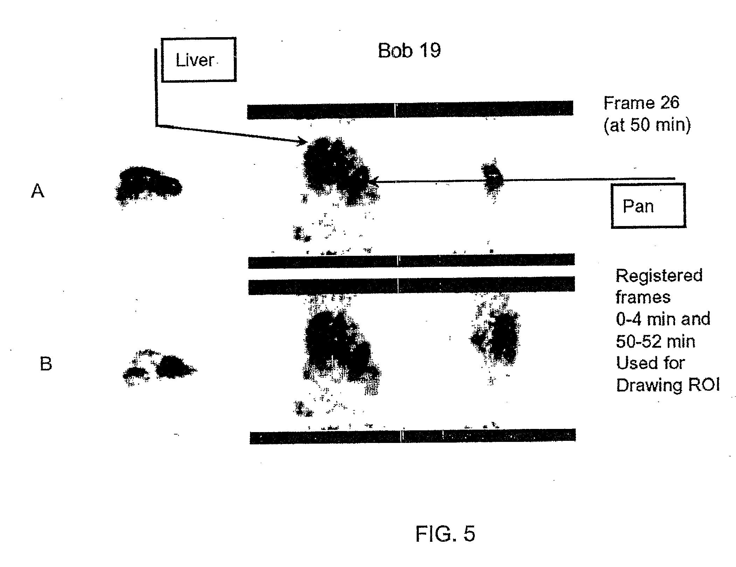

FIG. 5 shows images of Bob 19 liver and pancreas used for drawing regions of interest (ROI) (A) frame 26 at 50 minutes; (B) registered frames 0-4 minutes and 50-52 minutes.

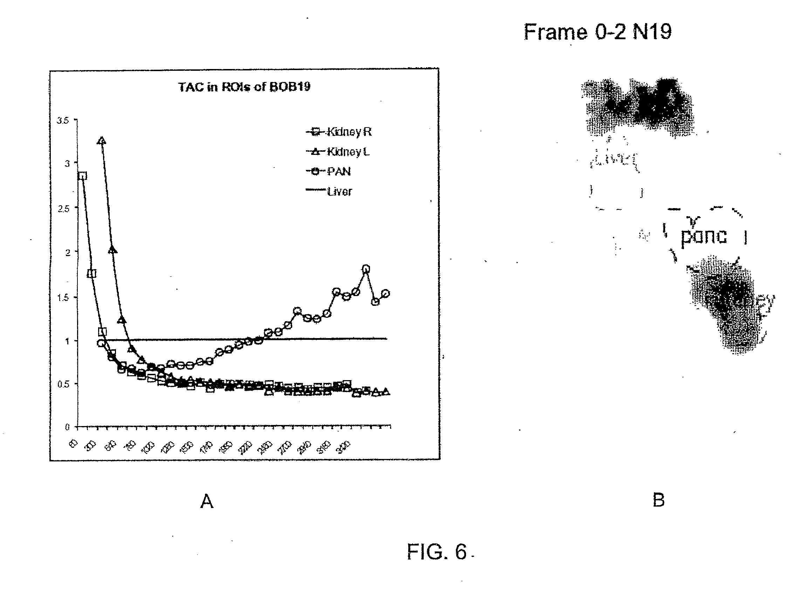

FIG. 6 depicts (A) time-activity-curve; and (B) ROI image for Bob 19.

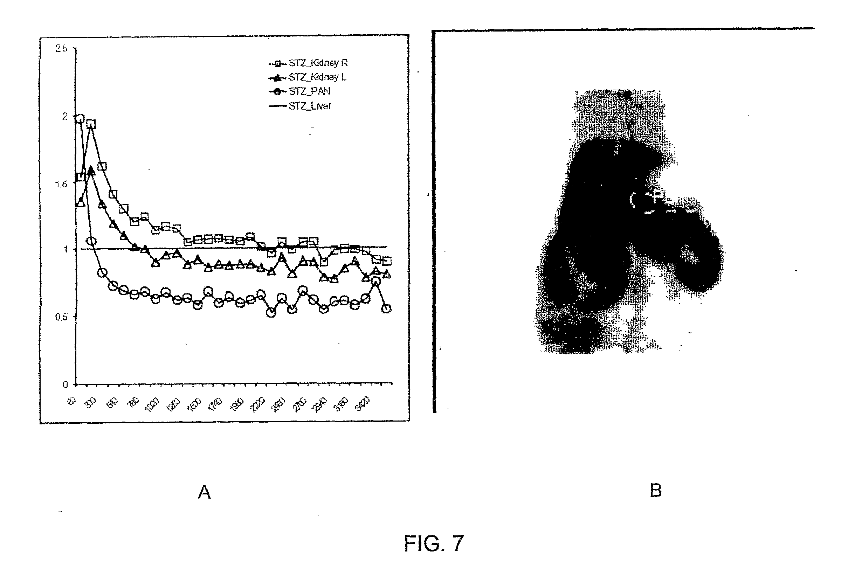

FIG. 7 shows (A) TAC and (B) ROI image (all 19 frames summed) for STZ treated diabetic Bob 19.

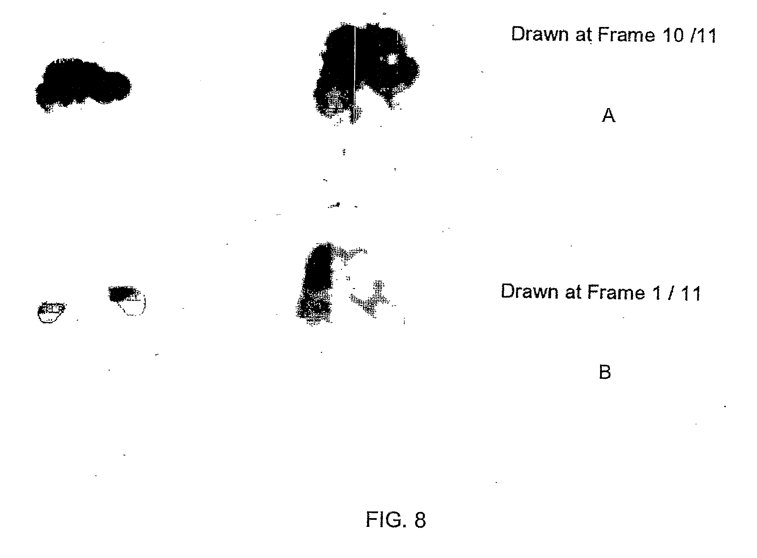

FIG. 8 shows ROIs for Bill 17 (A) drawn at frame 10/11; and (B) Drawn at frame 1/11.

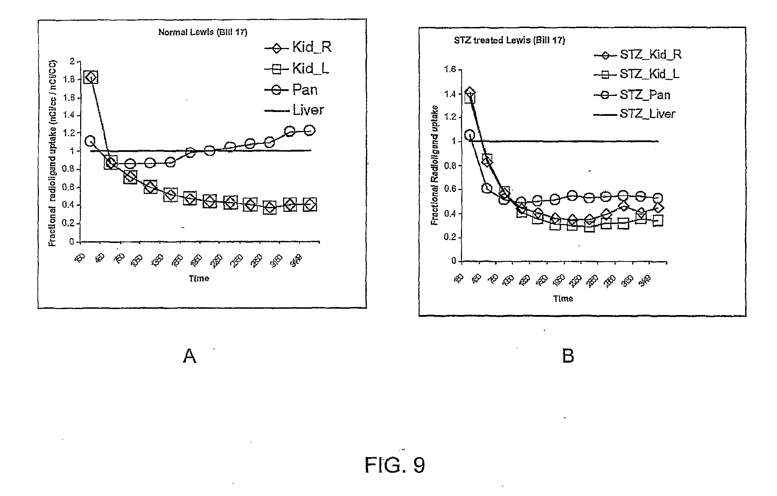

FIG 9 shows TACs for Bill 17 (A) before STZ; and (B) after STZ treatment.

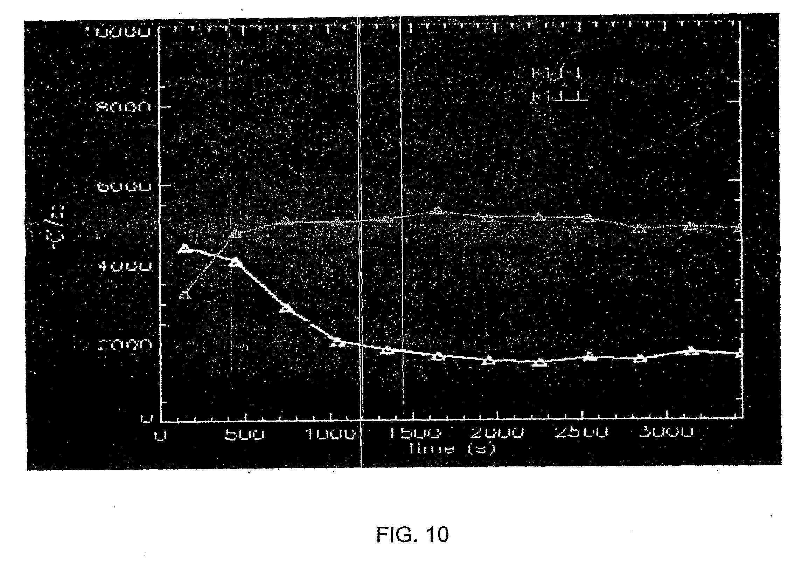

FIG. 10 shows TAC for STZ Bill 17.

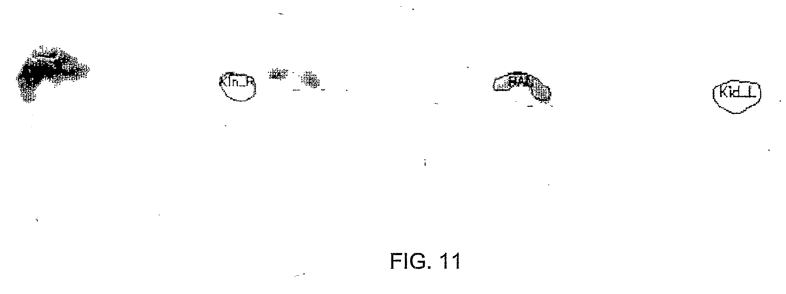

FIG. 11 depicts ROIs drawn on transverse sections moving rostral to caudal at different frames (times) in scan for Stu 16.

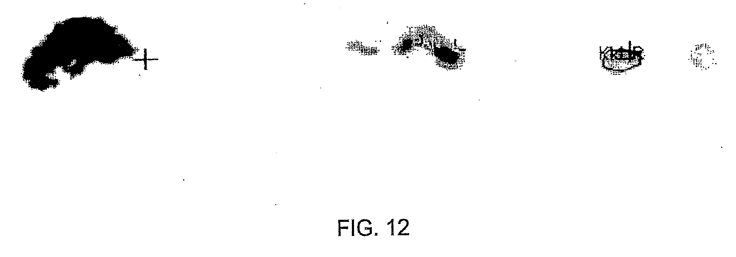

FIG. 12 shows ROIs for STZ treated diabetic Stu 16.

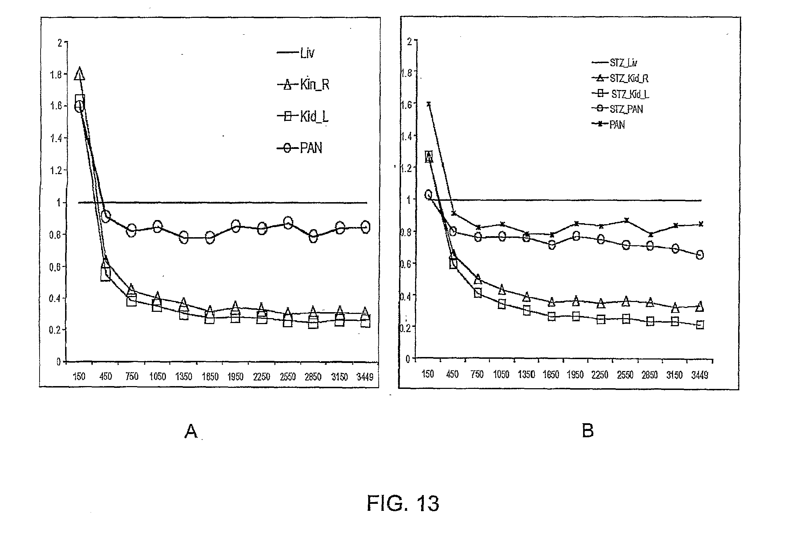

FIG. 13 shows TACs for Stu 16 (A) before; and (B) after STZ treatment.

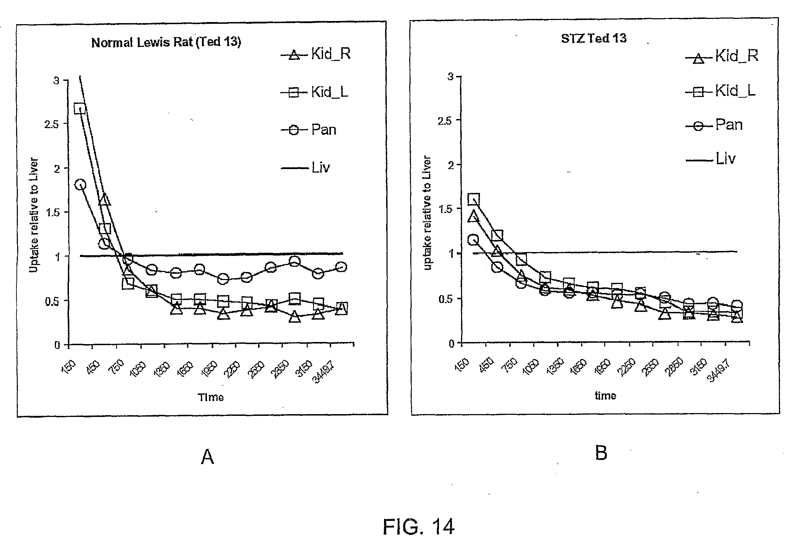

FIG. 14 shows TACs for Ted 13 (A) before; and (B) after STZ treatment.

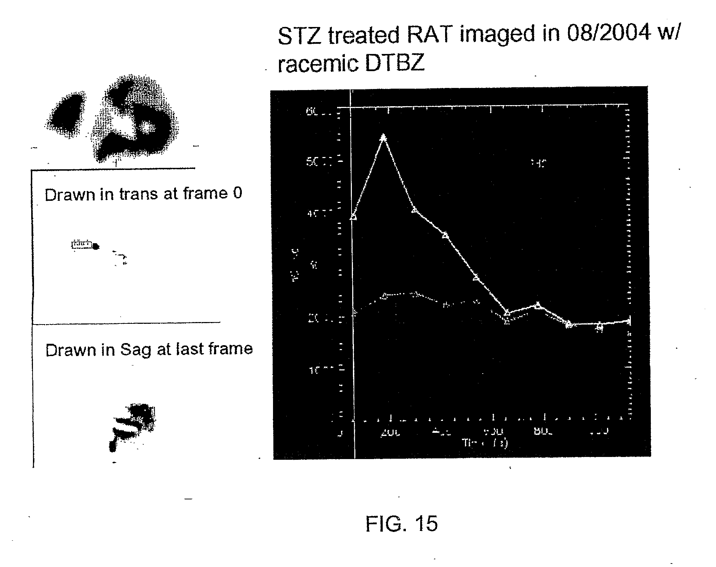

FIG. 15 shows ROIs and TAC of STZ treated rat imaged with racemic DTBZ.

FIG. 16 shows (A) pancreas in BB Rat A at beginning of study; and (B)-(C) at end of study.

FIG. 17 depicts ROI and TAC of spontaneously diabetic BB Rat A serial imaging on April 4, 2005.

FIG. 18 depicts ROI serial imaging of BB Rat A on April 11, 2005.

FIG. 19 shows TAC of BB Rat A on May 5, 2005.

FIG. 20 shows TAC of BB Rat A May 17, 2005.

FIG. 21 shows (A) standardized uptake versus (B) intraperitoneal glucose tolerance tests (IPGTT) for BB Rat A on April 4, 2005.

FIG. 22 shows (A) standardized uptake versus (B) IPGTT for BB Rat A on April 11, 2005.

FIG. 23 shows (A) standardized uptake versus (B) IPGTT for BB Rat A on April 20, 2005.

FIG. 24 shows (A) standardized uptake versus (B) IPGTT for BB Rat A on May 5, 2005.

FIG. 25 shows (A) standardized uptake versus (B) IPGTT for BB Rat A on May 17,2005.

### DETAILED DESCRIPTION OF THE INVENTION

As disclosed herein, the inventors have discovered non-invasive methods to readily visualize and measure beta cell mass of the endocrine pancreas using PET. The inventors have further discovered that this method provides a diagnostic test for nascent diabetes in individuals at risk, as well as providing support for therapies for metabolic and neuroendocrine disorders.

Accordingly, the present invention provides methods for determining the beta cell mass in a subject. As used herein, the "subject" is a mammal including, without limitation, a cow, dog, mouse, pig, rat, monkey or human. Preferably, the subject is human.

The present invention provides methods for determining the beta cell mass in the pancreas of a subject by administering to the subject an effective amount of a VMAT2-specific radioligand; obtaining one or more computerized image of at least a portion of the pancreas of the subject; and quantitatively analyzing the computerized image in order to determine the beta cell mass in the pancreas of the subj ect. In one embodiment of the present invention, the VNiAT2-specific radioligand is [11C] DTBZ. In another embodiment of the present invention, the computerized image is obtained using a positron emission tomography (PET). As used herein, "effective amount" refers to an amount of radioligand effective to provide an image of the region of interest using computer tomography. The amount of radioligand that is effective in providing an image will vary depending the particular factors of each case, including the type of radioligand, the type of subject, the subject's weight and the method of administration. These amounts can be readily determined by the skilled artisan.

In accordance with the method of the present invention, the radioligands disclosed herein may be administered to a human or animal subject by known procedures including, without limitation, parenteral administration (e.g. intravascular, intravenous, intraarterial, or parenchymatous administration). One preferred method of administration is parenteral administration, by venous or arterial injection.

For parenteral administration, the radioligand may be combined with a sterile aqueous solution, which is preferably isotonic with the blood of the subject. Such a formulation may be prepared by dissolving a solid active ingredient in water containing physiologically-compatible substances, such as sodium chloride, glycine, and the like, and having a buffered pH compatible with physiological conditions, so as to produce an aqueous solution, then rendering said solution sterile. The formulation may be presented in unit or multi-dose containers, such as sealed ampules or vials. The formulation also may be delivered by any mode of injection, including any of those described above.

The radioligand of the present invention also may be released or delivered from an osmotic mini-pump or other time-release device. The release rate from an elementary osmotic mini-pump may be modulated with a microporous, fast-response gel disposed in the release orifice. An osmotic mini-pump would be useful for controlling release, or targeting delivery, of the radioligand of choice.

The present invention also provides methods for diagnosing a metabolic or neuroendocrine disorder in a subject by administering to the subject an effective amount of a VMAT2-specific radioligand; obtaining one or more computerized image(s) of at least a portion of the pancreas of the subject; quantitatively analyzing the computerized image(s) in order to determine the beta cell mass in the pancreas of the subject; and comparing the beta cell mass with a baseline measure of beta cell mass, where a decreased beta cell mass or an increased beta cell mass versus the baseline measure is associated with the presence of a metabolic or neuroendocrine disorder. In one embodiment of the invention, the radioligand is [11C] DTBZ and the computerized image is obtained using PET. In a further embodiment of the invention, the metabolic disorder is a beta cell associated disorder including, but not necessarily limited to, an insulinoma or other neuroendocrine cancer. In another embodiment, the beta cell associated disorder is diabetes. In a particular embodiment of the invention, the metabolic disorder is type 1diabetes or type 2 diabetes. In another embodiment, the metabolic disorder is preclinical diabetes.

As used herein, "metabolic disorder" refers to any problem in the body that causes loss of metabolic homeostasis, and "beta cell associated disorder" refers to any disorder or disease characterized by changes in beta cell mass or function including, but not limited to, diabetes, preclinical diabetes and hypoglycemic disorders including insulinoma. As used herein, "diabetes" refers to any disorder of glucose metabolism leading to hyperglycemia and includes both type 1 and type 2 diabetes.

As used herein, "neuroendocrine disorder" refers to any disorder or disease involving or relating to the interaction between the nervous system and cellular hormone release, and includes, for example, Crohns disease and neuroendocrine cancers (NECs).

As used herein, "baseline measure" of BCM refers to a measure of BCM that is compared with a quantitative measure of BCM of the subject. By way of example, the baseline measure of BCM can be the BCM of a control subject. As used herein, "control subject" refers to a mammal including, without limitation, a cow, dog, mouse, pig, rat, monkey or human that does not have a metabolic disorder. In a preferred embodiment of the invention, the control subject is human. By way of further example, baseline measure of BCM may refer to the BCM of the subject measured at an earlier point in time.

Expected or normal BCM may also be determined by assaying the subject at a period of time in which the subject is asymptomatic of any disease or disorder. Expected or normal BCM may also be determined by assaying non-diseased subjects of a similar species age and of gender. For instance, BCM measurements may be obtained from at least 30 normal, healthy men between the ages of 25 and 40, to determine the normal BCM in adult males. A similar procedure may be followed to determine the normal BCM in females. Expected or normal BCM may also be determined by assaying the subject

Without limiting the invention, typically, for a quantitative diagnostic assay a positive result indicating the presence of a metabolic disorder is one in which the increase or decrease of BCM of the subject compared to the baseline measure of BCM is statistically significant. By way of non-limiting example, a statistically significant increase of BCM versus the control subject may be associated with the presence of an insulinoma, or other neuroendocrine cancer, or early type 2 diabetes. Similarly, a statistically significant decrease in BCM versus the baseline measure may be associated with the presence of diabetes.

The present invention additionally provides methods for assessing the prognosis of a subject at risk for developing type 1 or type 2 diabetes by periodically administering to the subject an effective amount of a VMAT2-specific radioligand; obtaining one or more computerized image(s) of at least a portion of the pancreas of the subject; quantitatively analyzing the computerized image(s) in order to determine the beta cell mass in the pancreas of the subject; and comparing the periodically determined beta cell mass with the baseline measure of beta cell mass, where a decreased beta cell mass versus the baseline measure is associated with the progression of preclinical diabetes to diabetes. In an embodiment of the invention, the radioligand is [11C] DTBZ and the computerized image is obtained using PET. The subject may be at risk for type 1 diabetes or type 2 diabetes.

By way of non-limiting example, the prognosis of a subject at risk for type 1 or type 2 diabetes could be monitored and assessed using the methods of the present invention by serially measuring the BCM of the subject (e.g. once per month for six months) and comparing each subsequent measurement to a previous BCM measurement (baseline measurement) or measurements of the same subject. A subject demonstrating a decrease in BCM compared to a previous (baseline) measurement may be further at risk of developing type 1 or type 2 diabetes.

The present invention also provides methods for determining the efficacy of a therapy for treating or preventing a metabolic disorder by periodically administering to the subject an effective amount of a VMAT2-specific radioligand; obtaining one or more computerized image(s) of at least a portion of the pancreas of the subject; quantitatively analyzing the computerized image(s) in order to determine the beta cell mass in the pancreas of the subject; and comparing the periodically determined beta cell mass with a baseline measure of beta cell mass, where a beta cell mass generally equivalent to the baseline measurement is indicative of successful therapy to treat or prevent the metabolic disorder.

As used herein "generally equivalent to the BCM of the control subject" means not significantly different from the baseline measurement. For example, a BCM that does not significantly differ from a particular baseline measure of BCM as determined by well-known statistical methods, could be said to be generally equivalent to the BCM of the control subject. In an embodiment of the invention, the radioligand is [11C] DTBZ and the computerized image is obtained using PET. In a further embodiment of the invention, the metabolic disorder is a pancreatic beta cell associated disorder. In a specific embodiment, the beta cell disorder is an insulinoma or other neuroendocrine tumor. In yet another specific embodiment, the beta cell disorder is diabetes. In still other embodiments of the invention, the beta cell disorder is type 1 diabetes, type 2 diabetes or preclinical type 1 diabetes.

The invention also provides methods for managing the treatment or prevention of diabetes by periodically administering to the subject an effective amount of a VMAT2-specific radioligand; obtaining one or more computerized image(s) of at least a portion of the pancreas of the subject; quantitatively analyzing the computerized image(s) in order to determine the beta cell mass in the pancreas of the subject; and comparing the periodically determined beta cell mass with a baseline measure of beta cell mass, where decreased beta cell mass versus the baseline measure is associated with the need for further therapy. In an embodiment of the invention, the radioligand is [11C] DTBZ and the computerized image is obtained using PET. In an embodiment, the diabetes is type 2 diabetes. In another embodiment, the diabetes is type I diabetes.

In accordance with the method of the present invention, the BCM of the subject or patient may be determined at any time following the initiation of therapy to treat or prevent diabetes. For example, BCM may be determined while the subject or patient is still undergoing treatment for diabetes.

Where the BCM of the subject or patient continues to remain decreased below normal, a physician may choose to continue with the subject's or patient's treatment for the diabetes. Similarly, where the BCM of the subject or patient does not noticeably increase or remain constant through successive assessments, it may be an indication that the treatment for diabetes is not working, and that treatment doses could be increased or otherwise altered.

On the other hand, where the BCM of the subject or patient remains constant or increases through successive assessments to approach normal or baseline BCM, it may be an indication that the treatment for diabetes is working and that treatment measures could be decreased or even ceased.

It is within the confines of the present invention to assess BCM following completion of a subject's or patient's treatment for diabetes, in order to determine whether the diabetes has recurred in the subject or patient. Accordingly, a BCM determination may provide a convenient way to conduct follow-ups of patients who have been diagnosed with diabetes. Furthermore, it is within the confines of the present invention to determine the BCM of a subject or patient as a means for determining the extent of diabetic disorder in the subject or patient and as a means for ascertaining appropriate treatment or prevention options.

The discovery that BCM can be quantitatively determined using [11C]BTBNZ and PET provides a means of identifying patients with diabetic disorders and presents the potential for commercial application in the form of a test for the diagnosis of diabetic conditions. The development of such a test provides general screening procedures. Such procedures can assist in the early detection and diagnosis of diabetic disorders and can provide a method for the follow-up of patients in whom there has been detection BCM decreased below normal. Similarly, the test also can assist in the early detection and diagnosis in patients with a beta cell associated disorder such as insulinoma or other neuroendocrine disorder and can provide a method of follow-up of patients in whom there has been detection of BCM increased above normal.

Accordingly, the present invention further provides kits for use in determining the beta cell mass in the pancreas of a subject and for diagnosing a metabolic or neuroendocrine disorder including diabetes in a subject comprising an effective amount of VMAT2-specific radioligand, a pharmaceutically acceptable carrier, and optionally, a PET scanner. In one embodiment, the.radioligand is [11C] DTBZ. However, any radioligand that specifically binds to VMAT2 can be used in the present invention, including, but not limited to any VMAT2-specific analogues of DTBZ. VMAT2-specific radioligands and DTBZ analogues can be synthesized by various methods well known to the skilled artisan.

In one embodiment of the invention the disorder is an insulinoma other neuroendocrine cancer or diabetes. In a preferred embodiment, the diabetes is type 1 or type 2 diabetes.

It is also within the confines of the present invention that a formulation containing a VMAT2-specific radioligand may be further associated with a pharmaceutically acceptable carrier, thereby comprising a pharmaceutical composition. Accordingly, the present invention further provides a pharmaceutical composition, comprising a VMAT2-specific radioligand and a pharmaceutically acceptable carrier. The pharmaceutically acceptable carrier must be "acceptable" in the sense of being compatible with the other ingredients of the composition, and not deleterious to the recipient thereof. Examples of acceptable pharmaceutical carriers include carboxymethyl cellulose, crystalline cellulose, glycerin, gum arabic, lactose, magnesium stearate, methyl cellulose, powders, saline, sodium alginate, sucrose, starch, talc and water, among others. Formulations of the pharmaceutical composition may be conveniently presented in unit dosage.

The pharmaceutical formulations of the present invention may be prepared by methods well known in the pharmaceutical arts. For example, the radioligand may be brought into association with a carrier or diluent, as a suspension or solution. Optionally, one or more accessory ingredients (e.g., buffers, flavoring agents, surface active agents and the like) also may be added. The choice of carrier will depend upon the route of administration. The pharmaceutical composition would be useful for administering the radioligand of the present invention to a subject. The radioligand would be provided in an amount that is effective to provide one or more image of a region of interest of the subject. That amount may be readily determined by the skilled artisan, as described above.

The present invention further provides method for diagnosing a neuroendocrine disorder in a subject comprising administering to the subject an effective amount of a vesicular monoamine transporter type 2 (VMAT2)-specific radioligand; obtaining at least one computerized image of at least a portion of a region of interest of the subject; quantitatively analyzing the computerized image in order to determine the beta cell mass in the region of interest of the subject; and comparing the beta cell mass with a baseline measure of beta cell mass, where a decreased beta cell mass or increased.beta cell mass versus the baseline measure is associated with the presence of a neuroendocrine disorder. In one embodiment of the invention, the neuroendocrine disorder is a neuroendocrine cancer, such as prostate cancer.

The present invention also provides methods for imaging a neuroendocrine tumor by administering to the subject an effective amount of a vesicular monoamine transporter type 2 (VMAT2)-specific radioligand; and obtaining at least one computerized image of at least a portion of a region of interest of the subject. In one embodiment, the tumor is a prostate tumor.

The present invention is described in the following Examples, which are set forth to aid in the understanding of the invention and should not be construed to limit in any way the scope of the invention as defined in the claims which follow thereafter.

### EXAMPLES

### Example 1

### Methods

### Radioligands

The stereochemically resolved (+)-9-O-desmethyl-a-dihydrotetrabenazine precursor of [11 C] DTBZ was obtained from ABX Advanced Biochemical Compounds (Radeberg, Germany). (+)-α-[11 C] DTBZ wassynthesized by [11C] methylation of the appropriate precursor and the product purified by HPLC (Jewett, et al, 1997, A simple synthesis of [11C]dihydrotetrabenazine (DTBZ), Nucl Med Biol 24:197-199, and Kilbourn, et al, 1995, Binding of alpha-dihydrotetrabenazine to the vesicular monoamine transporter is stereospecific, Eur J Pharmacol 278:249-252). The purity of [11C]-DTBZ preparations varied from 98.5 to 99.9 % of the desired (+) - product. Specific activities of carbon-11 labeled radiotracers were >2000 mCi/µmol at the time of injection.

### Diabetes induced by STZ

All animal studies were reviewed and approved by the Institutional Animal Care and Use Committee (IUCAC) at Columbia University's Medical School. All experiments were performed in accordance with the IACUC approved procedures. Diabetes mellitus was induced by a single intraperitoneal injection of streptozotocin (Sigma Aldrich, St. Louis, Mo) (50 mg/kg) to 250-350 g Lewis rats that had been fasted 4 hours to enhance the effectiveness of STZ treatment. STZ solution was prepared fresh by dissolving it in 0.1 M citrate buffer (pH 5.5) and terminally sterile filtered. Age and weight-matched control rats were injected with citrate buffer alone.

### Blood glucose and intraperitoneal glucose tolerance tests measurements

Blood samples were collected from the rat tail vein. The blood glucose (BG) levels of the rats were monitored daily using an Accu-Check blood glucose monitoring system (Roche Diagnostics, Somerville, NJ). Intraperitoneal glucose tolerance tests (IPGTT) were performed in fasting unanesthetized animals as previously described (Weksler-Zangen, et al, 2001, The newly inbred cohen diabetic rat: a non obese normolipidemic genetic model of diet-induced type 2 diabetes expressing sex differences, Diabetes 50:2521-2529). After baseline BG measurements, animals received an intraperitoneal injection of 1 g glucose/kg body wt. BG concentrations were measured again 5, 10, 15, 30, 60, and 120 min later. Animals were considered diabetic when four consecutive BG values were above 300 mg/dL and had abnormal IPGTT responses. Euglycemic control animals as well as diabetic rats meeting the above criteria were used.in imaging experiments. In some experiments, control rats were imaged, treated with STZ, determined to be diabetic and then re-imaged.

### Pancreas histology

Rat pancreata were dissected and fixed in 4% paraformaldehyde in PBS and embedded in paraffin. Ten µm-thick sections were obtained, deparaffinized and stained with hematoxylin and eosin. Sections were also stained with guinea pig anti bovine insulin antibodies or VMAT2 (Sigma-Aldrich, St. Louis, Mo) and developed by standard indirect immunohistochemistry methods. Sections were viewed with an optical Leica DME Microscope (Heidelberg, Germany) adapted with a digital photographic camera.

### PET scan study protocol

PET scans were performed on four normal and three diabetic adult rat subjects. Prior to imaging, the animals were anesthetized with intraperitoneal injections of ketamine and xylazine. After a whole-body transmission scan had been obtained (used to perform attenuation correction of the emission data), the radioligand was taken up in a sterile saline vehicle and 200-300 µCi of [11C] DTBZ was administered in a bolus injection via the penile vein. PET scans of the animals were acquired dynamically to 60 min postinjection on a Concorde microPET-R4 (CTI Molecular Imaging, Knoxville, TN, USA). The scanner provided a 100 x 80 mm field of view with a reconstructed resolution of 2.25 mm in the central 40 mm of the field of view. PET data were processed using attenuation correction matrix obtained by transmission scans and images were reconstructed using Fourier re-binning, followed by two-dimensional, filtered back projection.

### Data analysis and interpretation

Region of interest analysis and image reconstruction was performed with AsiPro (Concorde Microsystems, Knoxville, TN). Visual analysis was performed by 2 individuals experienced in PET interpretation using coronal, transverse and sagittal reconstructions. Reconstructed coronal PET images with a slice thickness of 5 mm were used to identify and measure the activity of each source organ of interest. Regions of interest (ROI) were drawn across image planes manually for the determination of time-activity curves. For the manual delineation of the ROI in the pancreas of normal rats, the inventors used the liver and stomach, as landmarks as well as the high avidity uptake of DTBZ in the β-cells of the islets of Langerhans. The region of interest in STZ-induced diabetic rats was drawn using liver and stomach landmarks and based on the pattern of DTBZ avidity seen in the pancreas of animals prior to treatment with STZ. The activity of [11C]DTBZ in the entire field and the ROI was estimated.

### Results and Discussion

Previous studies have shown that DTBZ specifically targets VMAT2 (Scherman, D. 1986, Dihydrotetrabenazine binding and monoamine uptake in mouse brain regions, J Neurochem 47:331-339, Scherman, et al., 1980, Effect of drugs on the ATP-induced and pH-gradient-driven monoamine transport by bovine chromaffin granules, Biochem Pharmacol 29:1883-1890, Scherman, et al., 1983, The catecholamine carrier of bovine chromaffin granules. Form of the bound amine, Mol Pharmacol 23:431-436, and Scherman, et al., 1983, Characterization of the monoamine carrier of chromaffin granule membrane by binding of [23H]dihydrotetrabenazine, Proc Natl Acad Sci USA 80:584-588). Using *in situ* hybridization, immunohistochemistry and confocal microscopy, (Anlauf, et al., 2003, Expression of the two isoforms of the vesicular monoamine transporter (VMAT1 and VMAT2) in the endocrine pancreas and pancreatic endocrine tumors, J Histochem Cytochem 51:1027-1040), and others (Maffei, et al., 2004, Identification of tissue-restricted transcripts in human islets, Endocrinology 145:4513-4521, and Weihe, et al., 1994, Localization of vesicular monoamine transporter isoforms (VMAT1 and VMAT2) to endocrine cells and neurons in rat, J Mol Neurosci 5:149-164) have shown that VMAT2 immunoreactivity co-localizes with insulin or is expressed with other β-cell markers and is absent from human islet cells stained with anti glucagon, somatostatin and pancreatic polypeptide. In the context of PET scanning with DTBZ, VMAT2 expression (as determined by immunohistochemistry in rodent tissues and by the inventors' own studies using quantitative real time PCR) is restricted to specific areas of the CNS, focal staining in the enteric nervous system, enterochromaffin cells, chromaffin cells of the adrenal medulla and in β-cells of the endocrine pancreas (Weihe, et al., 2000, Chemical neuroanatomy of the vesicular amine transporters, Faseb J 14:2435-2449). The neuropharmacology and neurofunctional anatomy of VMAT2 has been reviewed in detail (Weihe, et al., 2000, Chemical neuroanatomy of the vesicular amine transporters, Faseb J 14:2435-2449, and Henry, et al., 1998, The vesicular monoamine transporter: from chromaffin granule to brain, Neurochem Int 32:227246).

To model human T1 D a STZ treated Lewis rat model was selected. STZ is widely used to induce experimental diabetes in rodents (Wilson, et al., 1990, Streptozotocin interactions with pancreatic beta cells and the induction of insulin-dependent diabetes, Curr Top Microbiol Immunol 156:27-54). Previous studies have shown that STZ enters β-cells via the glucose transporter 2 and induces a series of intracellular changes, including formation of free radicals and liberation of nitric oxide, that results in β-cell death by necrosis (Szkudelski, T. 2001, The mechanism of alloxan and streptozotocin action in B cells of the rat pancreas, Physiol Res 50:537-546). Following STZ treatment, the Lewis rats used in these studies became stablely hyperglycemic.

The inventors next targeted VMAT2 expressed by β-cells of the endocrine pancreas with [11C] DTBZ, a radioligand suitable for PET scanning. Quantitative measurements of [11C] DTBZ uptake allowed the inventors to estimate VMAT2 density in the anatomical space occupied by the pancreas and indirectly, because VMAT2 is expressed only in beta cells of endocrine pancreas, obtain a measure of beta cell mass. PET imaging of normal rat pancreata showed that DTBZ uptake was concentrated around the central duct and uptake was higher in gastric and splenic regions of the pancreas compared to the head of the organ (Figure 2 panel A, C). Normal rat pancreata showed areas of DTBZ avidity in a distribution pattern that paralleled the previously reported size and density distributions of islets within the exocrine parenchyma of rats *(*Elayat, et al., 1995, An immunocytochemical and morphometric study of the rat pancreatic islets, J Anat 186 (Pt 3):629-637, and Bertelli, et al., 2001, Association between islets of Langerhans and pancreatic ductal system in adult rat. Where endocrine and exocrine meet together?, Diabetologia 44:575-584). Pancreatic DTBZ uptake increased monotonically from 1 to about 50 minutes post-injection during the 60 min imaging period.

PET imaging of the abdomen of the STZ induced diabetic rats showed differences both in the pattern and density of DTBZ uptake in the area of the pancreas compared to the non-diabetic control rodents (Figure 2 Panels B, D). Quantitative analysis of images reconstructed from the [11C] DTBZ PET scans suggests that the VMAT2 density in pancreata were reduced, but not completely ablated, in diabetic animals induced with STZ treatment relative to their pre-treatment levels and other control animals.

Reduced [11C] DTBZ uptake in the pancreas of STZ induced diabetic rats is consistent with the diabetogenic action of STZ, their abnormal IPGTT and the micro-anatomical findings obtained from review of sections of the pancreas obtained from rodents following imaging with [11C] DTBZ. Immunohistochemistry and H&E staining of sections of pancreata from STZ induced diabetic rats showed reduced islet area and frequencies compared to sections from control rodents (Figure 2, panels A-D). The frequency of cells with anti insulin and anti VMAT2 immunoreactivity within islets was also reduced in pancreata from STZ induced diabetic rats relative to the controls (Figure 2, Panels E-H).

### Example 2

### Quantitative Analysis of Beta Cell Mass Changes in Spontaneous Diabetes Rat Model Methods

### Radioligands

The stereochemically resolved (+)-9-O-desmethyl-a-dihydrotetrabenazine precursor of [11 C] DTBZ was obtained from ABX Advanced Biochemical Compounds (Radeberg, Germany). (+)-α-[11 C] DTBZ wassynthesized by [11C] methylation of the appropriate precursor and the product purified by HPLC. The purity of [11C]-DTBZ preparations varied from 98.5 to 99.9 % of the desired (+) - product. Specific activities of carbon-11 labeled radiotracers were >2000 mCi/µmol at the time of injection.

### The Spontaneous Rat Diabetes Model

All animal studies were reviewed and approved by the Institutional Animal Care and Use Committee (IUCAC) at Columbia University's Medical School. All experiments were performed in accordance with the IACUC approved procedures. BB Dp rates were obtained from Biomedical Research Models in Massachusetts. The BB-DP rat develops spontaneous diabetes (about 80%) between days 50 and 120 of life (mean 80 days).

### Blood glucose and intraperitoneal glucose tolerance tests measurements

Blood samples were collected from the rat tail vein. The blood glucose (BG) levels of the rats were monitored daily using an Accu-Check blood glucose monitoring system (Roche Diagnostics, Somerville, NJ). Intraperitoneal glucose tolerance tests (IPGTT) were performed in fasting unanesthetized animals as previously described (Weksler-Zangen, et al, 2001, The newly inbred cohen diabetic rat: a non obese normolipidemic genetic model of diet-induced type 2 diabetes expressing sex differences, Diabetes 50:2521-2529). After baseline BG measurements, animals received an intraperitoneal injection of 1 g glucose/kg body wt. BG concentrations were measured again 5, 10, 15, 30, 60, and 120 min later. Animals were considered diabetic when four consecutive BG values were above 300 mg/dL and had abnormal IPGTT responses. Animals were imaged at baseline before the development of diabetes at day 45. Following the baseline scan, animals were imaged at approximately two-week intervals until they showed clear sign of an abnormal glucose tolerance test.

### Pancreas histology

Rat pancreata were dissected and fixed in 4% paraformaldehyde in PBS and embedded in paraffin. Ten µm-thick sections were obtained, deparaffinized and stained with hematoxylin and eosin. Sections were also stained with guinea pig anti bovine insulin antibodies or VMAT2 (Sigma-Aldrich, St. Louis, Mo) and developed by standard indirect immunohistochemistry methods. Sections were viewed with an optical Leica DME Microscope (Heidelberg, Germany) adapted with a digital photographic camera.

### PET scan study protocol

PET scans were performed serially on the study subject at approximately day 50 and then every two weeks until the animals developed stable diabetes. Prior to imaging, the animals were anesthetized with intrapertioneal injections of ketamine and xylazine. After a whole-body transmission scan had been obtained (used to perform attenuation correction of the emission data), the radioligand was taken up in a sterile saline vehicle and 200-300 µCi of [11C] DTBZ was administered in a bolus injection via the penile vein. PET scans of the animals were acquired dynamically to 60 min post injection on a Concorde microPET-R4 (CTI Molecular Imaging, Knoxville, TN, USA). The scanner provided a 100 x 80 mm field of view with a reconstructed resolution of 2.25 mm in the central 40 mm of the field of view. PET data were processed using attenuation correction matrix obtained by transmission scans and images were reconstructed using Fourier re-binning, followed by two-dimensional, filtered back projection.

### Data analysis and interpretation

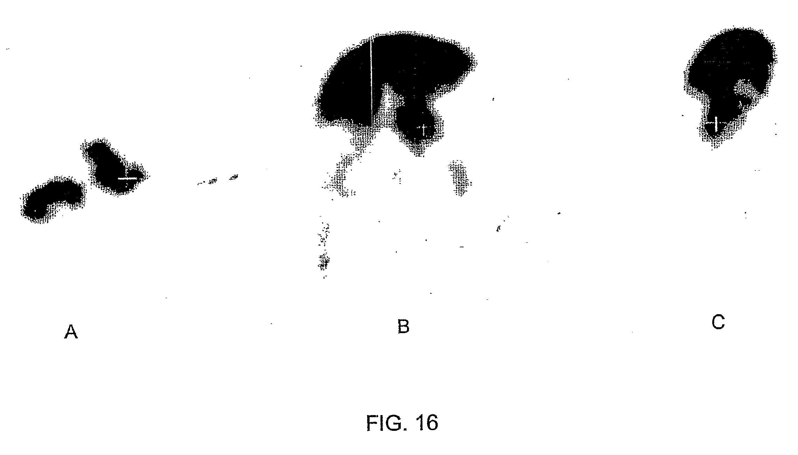

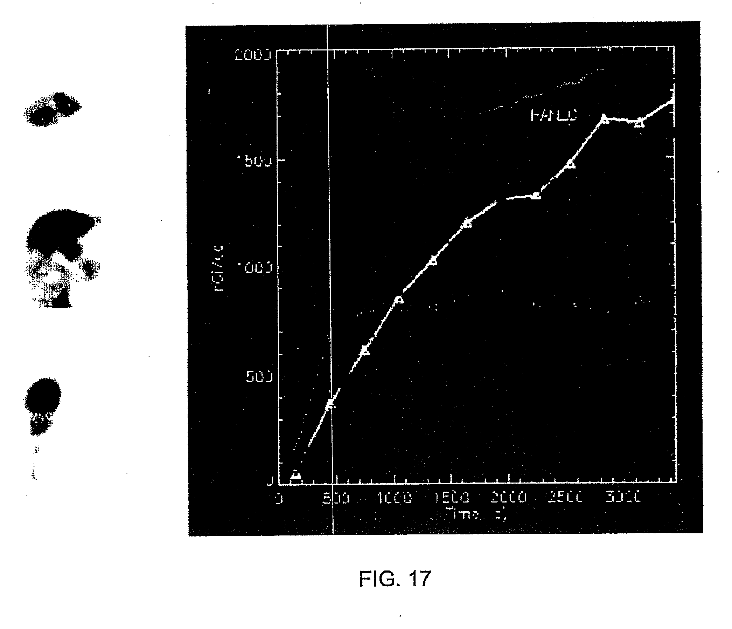

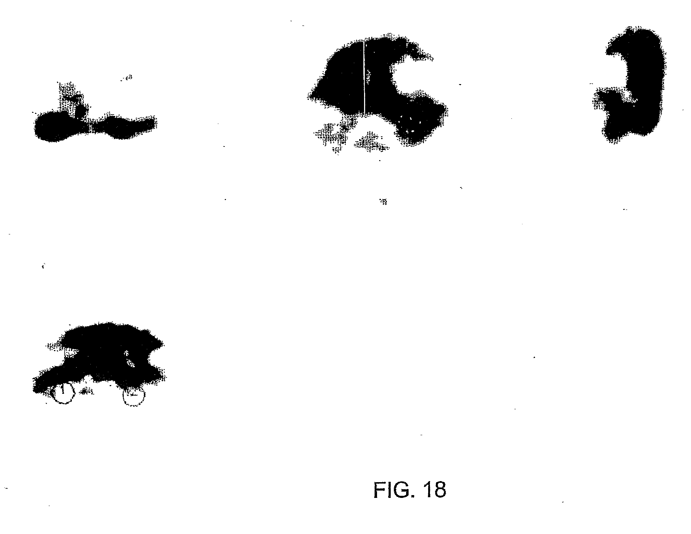

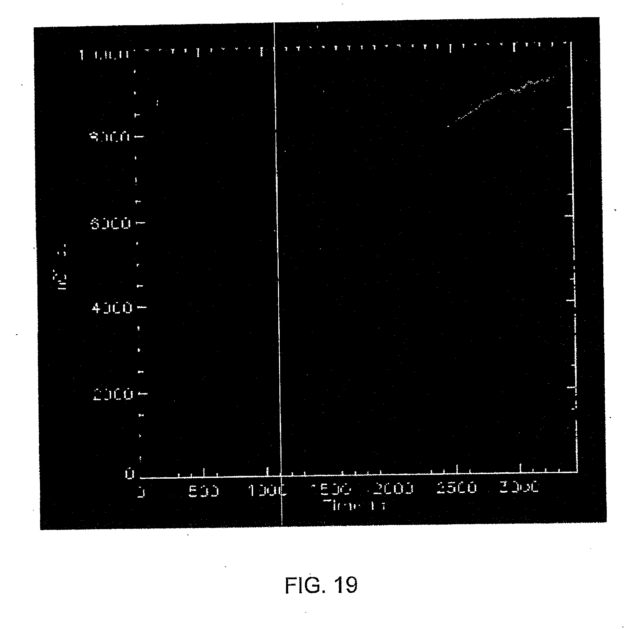

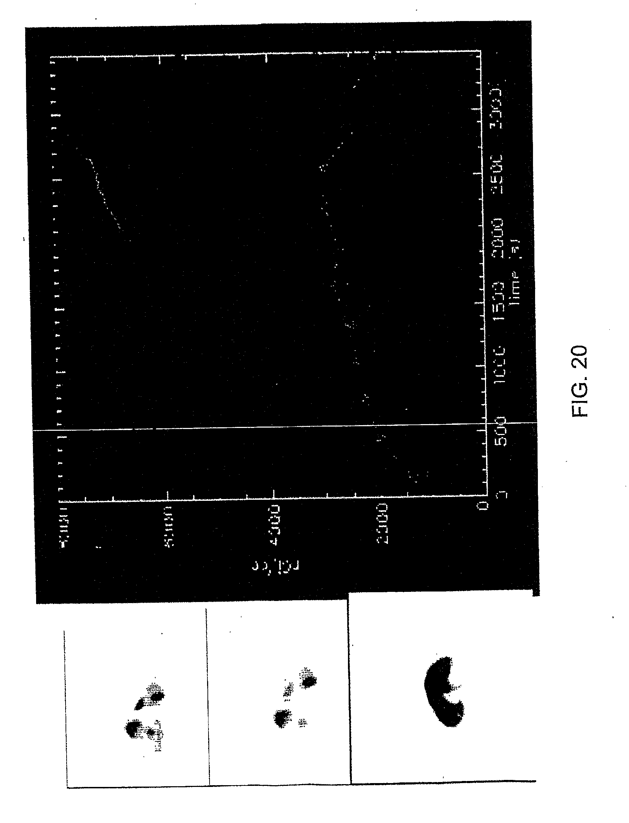

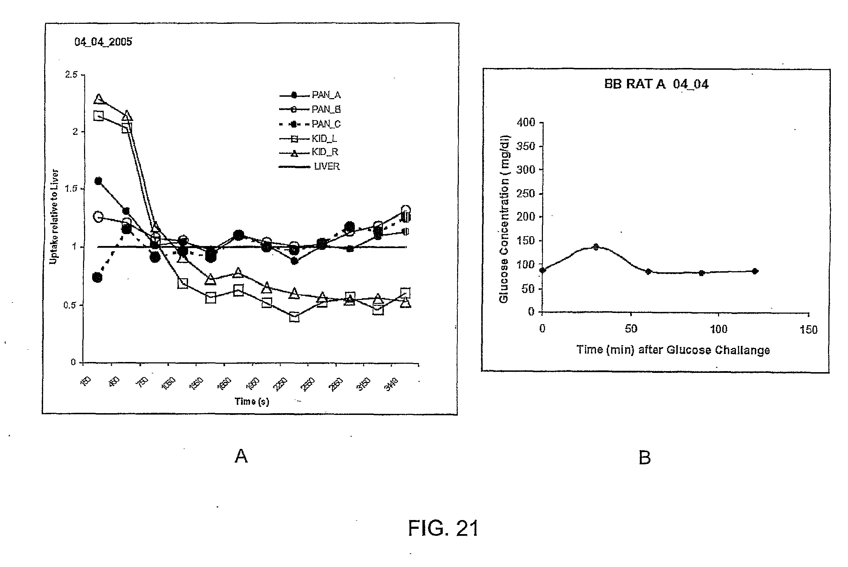

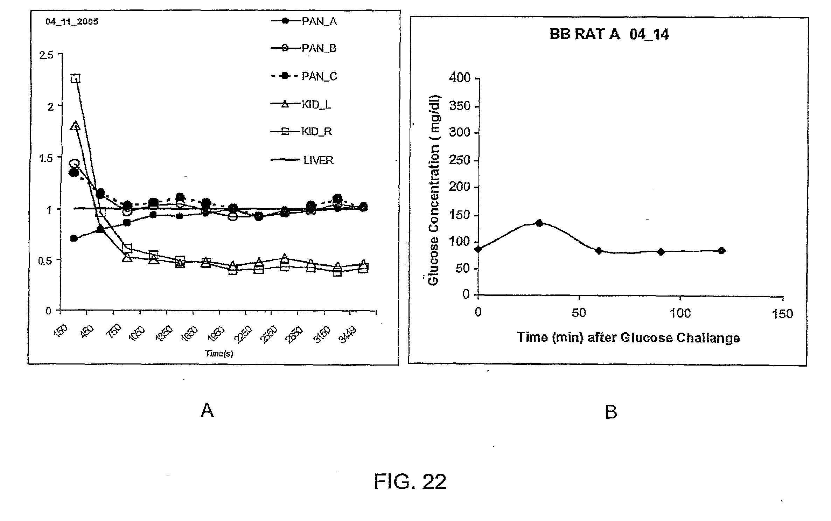

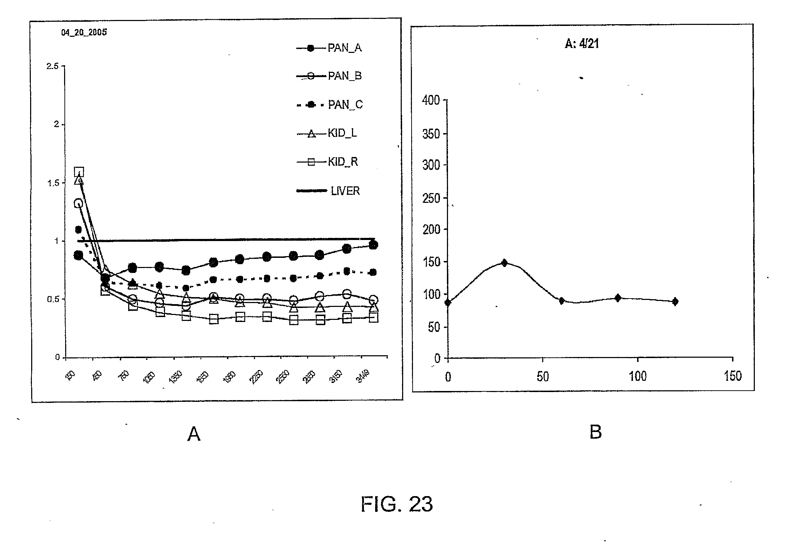

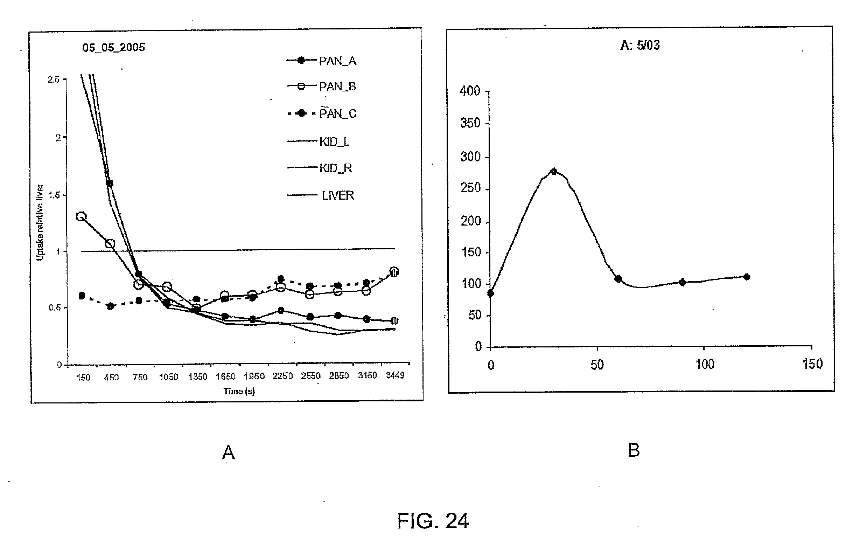

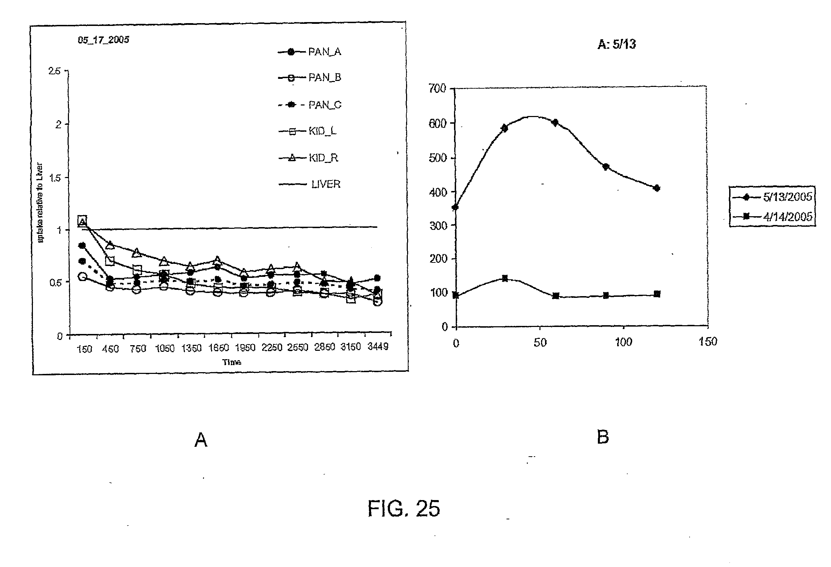

Region of interest analysis and image reconstruction was performed with AsiPro (Concorde Microsystems, Knoxville, TN). Visual analysis was performed by 2 individuals experienced in PET interpretation using coronal, transverse and sagittal reconstructions. Reconstructed coronal PET images with a slice thickness of 5 mm were used to identify and measure the activity of each source organ of interest. Regions of interest (ROI) were drawn across image planes manually for the determination of time-activity curves. For the manual delineation of the ROI in the pancreas, the inventors used the liver and stomach, as landmarks as well as the high avidity uptake of DTBZ in the β-cells of the islets of Langerhans. The region of interest in diabetic rats was drawn using liver and stomach landmarks and based on the pattern of DTBZ avidity seen in the pancreas of animals at baseline. The time versus activity curves of [11C]DTBZ uptake in the ROI was calculated using the ASIPro software (Figs. 16-25).

### Results and Discussion

PET scans with [11C]DTBZ in the BB-DP rat model, and the side-by-side comparison of quantitative data regarding the uptake of [11C]DTBZ within the endocrine pancreas with intraperitoneal glucose tolerance tests revealed that changes in uptake generally accompanied or preceded changes in glucose tolerance. In the series of ten BB-DP rats studied the inventors found that reductions of approximately 50% of [11C]DTBZ uptake resulted in signs of glucose intolerance and hyperglycemia. (Figs. 16-25).

### Example 3

### Determining Beta Cell Mass Changes in a Human Subject

### Radioligands

The stereochemically resolved (+)-9-O-desmethyl-a-dihydrotetrabenazine precursor of [11 C] DTBZ is obtained from ABX Advanced Biochemical Compounds (Radeberg, Germany). (+)-α-[11 C] DTBZ is synthesized by [11C] methylation of the appropriate precursor and the product purified by HPLC. The purity of [11C]-DTBZ preparations is from 98.5 to 99.9 % of the desired (+) - product. Specific activities of carbon-11 labeled radiotracers is >2000 mCi/µmol at the time of injection.

### PET scan study protocol

After a whole-body transmission scan has been obtained (used to perform attenuation correction of the emission data), the radioligand is taken up in a sterile saline vehicle and 200-300 mCi of [11C] DTBZ is administered intravenously. PET scans of the subject are acquired dynamically to 120 minutes post injection on a GE PET scanner. PET data are processed using attenuation correction matrix obtained by transmission scans and images were reconstructed using Fourier re-binning, followed by two-dimensional, filtered back projection.

### Data analysis and interpretation

Region of interest analysis and image reconstruction is performed with AsiPro (Concorde Microsystems, Knoxville, TN). Visual analysis is performed by an individual experienced in PET interpretation using coronal, transverse and sagittal reconstructions. Reconstructed coronal PET images with a slice thickness of 5 mm are used to identify and measure the activity of each source organ of interest. Regions of interest (ROI) are drawn across image planes manually for the determination of time-activity curves. For the manual delineation of the ROI in the pancreas of normal control subject, the liver and stomach are used as landmarks as well as the high avidity uptake of DTBZ in the β-cells of the islets of Langerhans.

The above process is repeated approximately every 28 days for three months and the change in BCM over time was recorded.

All publications, references, patents and patent applications cited herein are incorporated by reference in their entirety to the same extent as if each individual application, patent or patent application was specifically and individually indicated to be incorporated by reference in its entirety.

While the foregoing invention has been described in some detail for purposes of clarity and understanding, it will be appreciated by one skilled in the art, from a reading of the disclosure, that various changes in form and detail can be made without departing from the true scope of the invention in the appended claims.
Preferred embodiments of the invention are:
Item 1. A method for determining the beta cell mass in the pancreas of a subject comprising:
   (a) administering to the subject an effective amount of a vesicular monoamine transporter type 2 (VMAT2)-specific radioligand;
   (b) obtaining at least one computerized image of at least a portion of the pancreas of the subject; and
   (c) quantitatively analyzing the computerized image in order to determine the beta cell mass in the pancreas of the subject.
Item 2. The method of item 1, where the radioligand is [11C] DTBZ.
Item 3. The method of item 1, where the computerized image is obtained using a positron emission tomography (PET).
Item 4. The method of item 1, where the subject is a mammal.
Item 5. The method of item 1, where the subject is human.
Item 6. A method for diagnosing a metabolic disorder in a subject comprising:
   (a) administering to the subject an effective amount of a vesicular monoamine transporter type 2 (VMA T2)-specific radioligand;
   (b) obtaining at least one computerized image of at least a portion of the pancreas of the subject;
   (c) quantitatively analyzing the computerized image in order to determine the beta cell mass in the pancreas of the subject; and
   (d) comparing the beta cell mass with a baseline measure of beta cell mass, where a decreased beta cell mass or increased beta cell mass versus the baseline measure is associated with the presence of a metabolic disorder.
Item 7. The method of item 6, where the radioligand is [11C] DTBZ.
Item 8. The method of item 6, where the computerized image is obtained using a positron emission tomography (PET).
Item 9. The method of item 6, where the subject is a mammal.
Item 10. The method of item 6, where the subject is human.
Item 11. The method of item 6, where the disorder is a pancreatic beta cell associated disorder.
Item 12. The method of item 11, where the disorder is an insulinoma.
Item 13. The method of item 11, where the disorder is diabetes.
Item 14. The method of item 13, where the disorder is type 1 diabetes.
Item 15. The method of item 13, where the disorder is type 2 diabetes.
Item 16. The method of item 6, where the disorder is preclinical type 1 diabetes.
Item 17. A method for assessing the prognosis of a subject at risk for developing diabetes comprising periodically:
   (a) administering to the subject an effective amount of a vesicular monoamine transporter type 2 (VMA T2)-specific radioligand;
   (b) obtaining at least one computerized image of at least a portion of the pancreas of the subject;
   (c) quantitatively analyzing the computerized image in order to determine the beta cell mass in the pancreas of the subject; and
   (d) comparing the periodically determined beta cell mass with a baseline measure of beta cell mass, where decreased beta cell mass versus the baseline measure is associated with the progression of preclinical diabetes to diabetes.
Item 18. The method of item 17, where the radioligand is [11C] DTBZ.
Item 19. The method of item 17, where the computerized image is obtained using a positron emission tomography (PET).
Item 20. The method of item 17, where the subject is a mammal.
Item 21. The method of item 17, where the subject is human.
Item 22. A method for determining the efficacy of a therapy for treating or preventing a metabolic disorder comprising periodically:
   (a) administering to the subject an effective amount of a vesicular monoamine transporter type 2 (VMAT2)-specific radioligand;
   (b) obtaining at least one computerized image of at least a portion of the pancreas of the subject;
   (c) quantitatively analyzing the computerized image in order to determine the beta cell mass in the pancreas of the subject; and
   (d) comparing the periodically determined beta cell mass with a baseline measure of beta cell mass, where a beta cell mass generally equivalent to the baseline measure, is indicative of a successful therapy to treatment or prevention of the metabolic disorder.
Item 23. The method of item 22, where the radioligand is [11C] DTBZ.
Item 24. The method of item 22, where the computerized image is obtained using a positron emission tomography (PET).
Item 25. The method of item 22, where the subject is a mammal.
Item 26. The method of item 22, where the subject is human.
Item 27. The method of item 22, where the disorder is a pancreatic beta cell associated disorder.
Item 28. The method of item 22, where the disorder is an insulinoma.
Item 29. The method of item 22, where the disorder is diabetes.
Item 30. The method of item 29, where the disorder is type 1 diabetes.
Item 31. The method of item 22, where the disorder is preclinical type 1 diabetes.
Item 32. A method for managing the treatment or prevention of diabetes comprising periodically:
   (a) administering to the subject an effective amount of a vesicular monoamine transporter type 2 (VMA T2)-specific radioligand;
   (b) obtaining at least one computerized image of at least a portion of the pancreas of the subject;
   (c) quantitatively analyzing the computerized image in order to determine the beta cell mass in the pancreas of the subject; and
   (d) comparing the periodically determined beta cell mass with a baseline measure of beta cell mass, where a decreased beta cell mass versus the baseline measure is associated with the need for further therapy.
Item 33. The method of item 32, where the radioligand is [11C] DTBZ.
Item 34. The method of item 32, where the computerized image is obtained using a positron emission tomography (PET).
Item 35. The method of item 32, where the subject is a mammal.
Item 36. The method of item 32, where the subject is human.
Item 37. The method of item 32, where the diabetes is type 1 diabetes.
Item 38. A kit for use in determining the beta cell mass in the pancreas of a subject comprising an effective amount of a vesicular monoamine transporter type 2 (VMA T2)-specific radioligand, a pharmaceutically acceptable carrier, and optionally a PET scanner.
Item 39. The kit of item 38, where the radioligand is [11C] DTBZ.
Item 40. The kit of item 39, where the subject is a mammal.
Item 41. The kit of item 38, where the subject is human.
Item 42. A method for diagnosing a neuroendocrine disorder in a subject comprising:
   (a) administering to the subject an effective amount of a vesicular monoamine transporter type 2 (VMAT2)-specific radioligand;
   (b) obtaining at least one computerized image of at least a portion of a region of interest of the subject;
   (c) quantitatively analyzing the computerized image in order to determine the beta cell mass in the region of interest of the subject; and
   (d) comparing the beta cell mass with a baseline measure of beta cell mass, where a decreased beta cell mass or increased beta cell mass versus the baseline measure is associated with the presence of a neuroendocrine disorder.
Item 43. The method of item 42, where the neuroendocrine disorder is a neuroendocrine cancer.
Item 44. A method for imaging a neuroendocrine tumor comprising:
   (a) administering to the subject an effective amount of a vesicular monoamine transporter type 2 (VMA T2)-specific radioligand; and
   (b) obtaining at least one computerized image of at least a portion of a region of interest of the subject.
Item 45. The method of item 43, where the tumor is a prostate tumor.

## Claims

1. A pharmaceutical composition for use in determining a beta cell mass in the pancreas of a subject, comprising: an effective amount of a VMAT2-specific radioligand and a pharmaceutically acceptable carrier.

2. A pharmaceutical composition for use in diagnosing a metabolic disorder or a neuroendocrine disorder in a subject based on the beta cell mass in the pancreas of the subject, the pharmaceutical comprising comprising: an effective amount of a VMAT2-specific radioligand and a pharmaceutically acceptable carrier.

3. The pharmaceutical composition of claim 1 or 2, wherein the radioligand is [11C] DTBZ or a VMAT2-specific analogue of DTBZ.

4. The pharmaceutical composition of claim 1 or 2, wherein the radioligand is [11C] DTBZ.

5. The pharmaceutical composition of any of claims 1-4, wherein the subject is a mammal.

6. The pharmaceutical composition of any of claims 1-5, wherein the subject is human.

7. The pharmaceutical composition of any of claims 2-6, wherein the metabolic disorder is a pancreatic beta cell associated disorder.

8. The pharmaceutical composition of claim 7, where the metabolic disorder is an insulinoma.

9. The pharmaceutical composition of claim 7, where the metabolic disorder is type 1 diabetes.

10. The pharmaceutical composition of claim 7, where the metabolic disorder is type 2 diabetes.

11. The pharmaceutical composition of claim 7, wherein the metabolic disorder is preclinical diabetes.

12. The pharmaceutical composition of any of claims 2-6, wherein the neuroendocrine disorder is a neuroendocrine cancer.

13. The pharmaceutical composition of any of claims 2-6, wherein the neuroendocrine disorder is a prostate cancer.

14. The pharmaceutical composition of any of claims 1-13, wherein the pharmaceutical composition is in a unit dosage form.
